# EUROPEAN PATENT APPLICATION

(11) **EP 4 202 018 A1**
(43) Date of publication of application: **28.06.2023**
(21) Application number: 21217615.0
(22) Date of filing: 23.12.2021
(51) Int. Cl.: C10G 2/00

(54) **FISCHER-TROPSCH SYNTHESIS STARTUP**

(71) Applicant: BP P.L.C., London SW1Y 4PD (GB); Johnson Matthey Davy Technologies Limited, London EC4A 4AB (GB)
(72) Inventor: The designation of the inventor has not yet been filed
(74) Representative: Hamer, Christopher K.

(57) **Abstract**

The present disclosure relates generally to processes for initiating Fischer-Tropsch synthesis.

In particular, the application concerns a process for the initiation of Fischer-Tropsch synthesis in a reaction zone having a catalyst disposed therein, the catalyst being in a reduced form, the process comprising: (i) providing the reaction zone with a temperature of no more than 140 °C; then (ii) purging the reaction zone with a purge gas comprising N₂ at a pressure in the range of 2 barg to 10 barg; then (iii) contacting the catalyst in the reaction zone with a gaseous reaction mixture comprising H₂ and CO in a ratio of between 1:1 and 3:1 at a pressure of no more than 20 barg and at a temperature of no more than 140 °C; then (iv) heating the reaction zone to a temperature of at least 200 °C; and (v) pressurizing the reaction zone to a pressure in the range of 30 barg and 45 barg.

## Description

### BACKGROUND OF THE DISCLOSURE

### FIELD

The present disclosure relates to Fischer-Tropsch processes, specifically to techniques for initiating Fischer-Tropsch processes.

### TECHNICAL BACKGROUND

The conversion of synthesis gas (i.e., a mixture of carbon monoxide and hydrogen, also known as syngas) into hydrocarbons by the Fischer-Tropsch process has been known for decades, but has historically lagged in performance compared to other hydrocarbon synthesis techniques. The growing importance of alternative energy sources has resulted in renewed interest in the Fischer-Tropsch (FT) process as it allows a direct and environmentally acceptable route to high-quality fuels and feedstock chemicals.

FT processes are known for producing linear hydrocarbons for use in fuels, as well as oxygenates which can be useful in fuels and also serve as valuable feedstock chemicals. The hydrocarbon fuel derived from FT processes is typically better able to meet increasingly stringent environmental regulations compared to conventional refinery-produced fuels, as FT-derived fuels typically have lower contents of sulfur, nitrogen, and aromatic compounds, which contribute to the emission of potent pollutants such as SO₂, NOₓ, and particulates. Alcohols derived from FT processes often have a higher octane rating than hydrocarbons and thus burn more completely, thereby reducing the environmental impact of such a fuel. Alcohols and other oxygenates obtained may also be used as reagents in other processes, such as in the synthesis of lubricants.

A variety of transition metals have been identified to be catalytically active in the conversion of synthesis gas into hydrocarbons and oxygenated derivatives thereof. In particular, cobalt, nickel, and iron have been studied, often in combination with a support material, of which the most common are alumina, silica and carbon.

In the typical preparation of supported cobalt-containing FT synthesis catalysts, a solid support material is contacted with a solution of a soluble cobalt compound, such as cobalt nitrate. The impregnated support is subsequently calcined and/or oxidized to form a cobalt oxide, typically one or more of CoO, Co₂O₃, or Co₃O₄. However, such oxides typically have poor FT catalytic activity and must be reduced to form the preferred catalytically-active species of cobalt metal.

During startup, the reduced catalyst is contacted in the FT reactor with syngas at elevated temperatures and/or pressures, until a desired steady-state operation is achieved. During the startup, the reaction efficiency can be undesirably low, and the product mix can be generally different than that from steady-state operation. After startup, i.e., once the desired steady-state operation is achieved, the reactor is continually operated in steady-state conditions for long periods of time. As startup conditions can vary significantly from steady-state conditions, poor reactor control can result in inefficient energy use, poor product distribution, and even damage to the reactor or catalyst.

Accordingly, there exists a need to develop improved protocols for the startup of Fischer-Tropsch synthesis reactors.

### SUMMARY

The inventors have found processes to initiate Fischer-Tropsch synthesis that afford excellent control of reactor conditions such as pressure and temperature. This results in increased process efficiency during start-up and prevents the occurrence of dangerous thermal runaway due to the exothermic contact of syngas with fresh catalyst.

Thus, in one aspect, the present disclosure provides a process for the initiation of Fischer-Tropsch synthesis in a reaction zone having a catalyst disposed therein, the catalyst being in a reduced form, the process comprising:
(i) providing the reaction zone with a temperature of no more than 160 °C; then
(ii) purging the reaction zone at a temperature of no more than 160 °C with a purge gas comprising N₂, at a pressure in the range of 2 barg to 15 barg; then
(iii) contacting the catalyst in the reaction zone with a gaseous reaction mixture comprising H₂ and CO in a ratio of in the range of 1:1 to 3:1 at a pressure of no more than 45 barg and at a temperature of no more than 160 °C; then, while in contact with the gaseous reaction mixture,
(iv) heating the reaction zone to a temperature of at least 200 °C; and
(v) pressurizing the reaction zone to a pressure in the range of 25 barg to 45 barg.

In various embodiments of the processes of the present disclosure, the heating of step (iv) is conducted with a plurality of different heating rates, comprising:
(a) heating at a rate in the range of 10 °C/hr to 60 °C/hr at temperatures below 160 °C;
(b) heating at a rate in the range of 2 °C/hr to 30 °C/hr at temperatures in the range of 160 °C to 180 °C; and
(c) heating at a rate in the range of 0.5 °C/hr to 7 °C/hr at temperatures in the range of 180 °C to 220 °C.

Other aspects of the disclosure will be apparent to those skilled in the art in view of the description that follows.

### BRIEF DESCRIPTION OF THE DRAWINGS

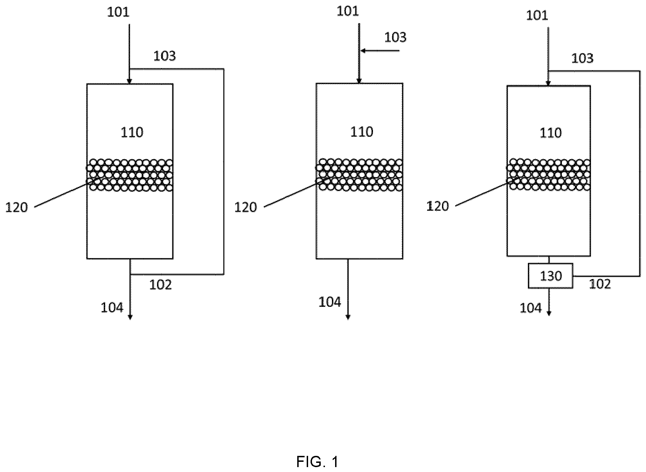

Fig. 1 is a schematic depiction of a reactors suitable for use in the processes described herein. Reactor 110 is charged with catalyst 120. Gas (e.g., CO, H₂, inerts) is introduced through inlet 101, and optionally run as a once -through configuration, exiting the system at 104 and hydrocarbon products are removed through outlet 104 for further processing. Unreacted gas are optionally removed via separator 130 through recycle outlet 102, and optionally recycled back into inlet 101 through recycle feed 103.

### DETAILED DESCRIPTION

The present disclosure relates to techniques for initiating Fischer-Tropsch synthesis processes in which mixtures of hydrogen and carbon monoxide are converted to hydrocarbons. In a typical Fischer-Tropsch process, a supported cobalt oxide catalyst precursor is reduced with hydrogen at elevated temperature (typically above 300 °C). If the reduction is performed ex *situ*, the catalyst is transferred under inert conditions to the reactor. If an *in situ* reduction is performed, the catalyst is cooled to Fischer-Tropsch synthesis reaction temperature and then the catalyst is contacted with syngas. A flow of syngas (typically a mixture of H₂ and CO, optionally with CO₂ and with nitrogen as a diluent) is provided to the reactor to start up the reaction process. In the startup, the temperature and pressure are raised to provide the ultimately desired reaction conditions, which are typically maintained throughout the process.

The present inventors have found that contacting a freshly-reduced Fischer-Tropsch catalyst with syngas can lead to a dangerous exotherm as carbon monoxide and hydrogen are exothermically converted to hydrocarbons. Over time, the catalyst typically loses the activity associated with a freshly reduced catalyst, and the process enters a steady-state regime in which the reaction rate is slower and less heat is generated. Unless mitigated, the exotherm during start-up can result in a dangerous increase in temperature in the Fischer-Tropsch reactor, leading to potential damage to the reactor and to the catalyst as well as an undesirable waste of energy.

One way to curb this exotherm is the introduction of a syngas that has a significant proportion of an inert gas, such as N₂ gas. The proportion of syngas can be low initially and then slowly increased as the catalyst loses activity and transitions from the start-up operating regime to the steady state operating regime. This approach of ramping the syngas concentration is reasonable for small, experimental-scale reactors. However, in a full-scale industrial Fischer-Tropsch plant, this solution is less workable. At large scale, a compressor capable of handling over 5000 Nm3/m3.h at pressures in excess of 10 barg is utilized. Converted into absolute terms, over 20m3 of catalyst used in a typical reactor of 1000 reactor tubes. Critically, compressors operating at such extreme conditions have narrow design windows with respect to operating pressure, volumetic flow rate, , developed head (differential pressure) and operating fluid composition. GHSV over the catalyst will be related to the volumetric flow achieved through a recycle compressor. Further, typical large-scale reactors have a recycle mechanism that allows re-introduction of light hydrocarbons and unreacted syngas to the reactor feed. The use of a low proportion of syngas results in a very low recycle throughput, and can result in impaired compressor performance. As such, the present inventors have found that it is generally preferable to maintain a constant syngas proportion throughout the start-up and steady-state regimes with fixed reactor feed gas composition and inerts level and flowrate to ensure stable operation and heat removal as we start up to avoid runaways.

Accordingly, the present inventors have sought an alternative start-up procedure for initiating Fischer-Tropsch synthesis that can avoid substantially changing of the syngas feed and also avoids the creation of a dangerous exotherm.

Thus, in one aspect, the present disclosure provides a process for the initiation of Fischer-Tropsch synthesis in a reaction zone having a reduced catalyst disposed therein, the process comprising:
(i) providing the reaction zone with a temperature of no more than 160 °C; then
(ii) purging the reaction zone at a temperature of no more than 160 °C with a purge gas comprising N₂, at a pressure in the range of 2 barg to 15 barg; then
(iii) contacting the catalyst in the reaction zone with a gaseous reaction mixture comprising H₂ and CO in a ratio of in the range of 1:1 to 3:1 at a pressure of no more than 45 barg and at a temperature of no more than 160 °C; then, while in contact with the gaseous reaction mixture,
(iv) heating the reaction zone to a temperature of at least 200 °C; and
(v) pressurizing the reaction zone to a pressure in the range of 25 barg to 45 barg.

As described in more detail below, a variety of reaction systems can be used in performing the processes described herein, and the person of ordinary skill in the art will adapt conventional systems as necessary. Suitable means for controlling the reactor temperature during activation and start-up can be used, as known by those skilled in the art; including electrical heating, direct heating of the reactor tubes with steam, or indirect heating with steam via an eductor or jet pump arrangement to heat a circulating-water circuit around the reactor tubes. An example of a reaction system for use in performing the processes of the disclosure is shown in schematic view in FIG. 1. Reactor 110 is charged with reduced catalyst 120. Syngas is introduced through inlet 101, and hydrocarbon products are removed through outlet 104 for further processing. Unreacted syngas, and optionally light hydrocarbons, are removed through recycle outlet 102, and recycled back into inlet 101 through recycle feed 103.

Thus, a reaction zone (e.g., including a reactor 110 as described above) has a reduced catalyst disposed therein (e.g., reduced catalyst 120 as described above). The person of ordinary skill in the art can provide a reduced catalyst using conventional methods in the art, e.g., by reducing an oxidic catalyst precursor with hydrogen. Such reduction can be performed in situ, i.e., in the reactor in which the subsequent Fischer-Tropsch synthesis is to be performed, or can be performed ex situ, i.e., in a separate reactor, and loaded into the FT reactor, desirably under inert conditions.

As noted above, the reaction zone having the reduced catalyst disposed therein is provided with a temperature of no more than 140 °C. In cases where the catalyst is reduced ex situ, the catalyst will often be no warmer than 140 °C when introduced to the reaction zone. However, in cases where the reaction zone having the reduced catalyst therein is in excess of 140 °C, e.g., when the catalyst is reduced in situ, the reaction zone can be cooled down to a temperature of no more than 140 °C. For example, in various embodiments as otherwise described herein, providing the reaction zone includes cooling the reaction zone from a temperature of at least 150 °C to a temperature of no more than 140 °C. In various embodiments as otherwise described herein, the reaction zone in step (i) is cooled from a temperature of at least 160 °C (e.g., at least 170 °C, or at least 180 °C, or at least 190 °C, or at least 200 °C).

When there is cooling in step (i), it can be performed in any desirable, manner, e.g., using a variety of gases. In various embodiments as otherwise described herein, the cooling occurs in the presence of a gaseous mixture comprising H₂ and N₂. In certain embodiments as otherwise described herein, the gaseous mixture comprises at least 5% H₂. For example, the gaseous mixture may comprise at least 10% H₂, or at least 15% H₂, or at least 25% H₂. In certain embodiments, the gaseous mixture in the cooling step comprises less than 5% CO, for example less than 1% CO or less than 0.1% CO or substantially no CO.

The reaction zone in step (i) can be provided at a variety of temperatures no more than 140 °C. For example, in various embodiments as otherwise described herein, the temperature of the reaction zone in step (i) is no more than 130 °C, e.g., no more than 120 °C. In various embodiments as otherwise described herein, the reaction zone in step (i) is at a temperature in the range of 80 °C to 140 °C. For example, in various particular such embodiments, the reaction zone in step (i) is at a temperature in the range of 80 °C to 130 °C, or 80 °C to 120 °C. In various particular such embodiments, the reaction zone in step (i) is at a temperature in the range of 100 °C to 140 °C, for example 100 °C to 130 °C, or 100 °C to 120 °C. In various particular such embodiments, the reaction zone in step (i) is at a temperature in the range of 80 °C to 160 °C, for example 110 °C to 130 °C.

The reaction zone is then purged with a purge gas comprising N₂, at a pressure in the range of 2 barg to 15 barg. The purge gas operates to remove hydrogen and any volatile reduction products or water from the reduced catalyst before it is contacted it with syngas. In various embodiments as otherwise described herein, the purge gas comprises at least 90% N₂, for example, at least 95% N₂, or at least 99% N₂, or is substantially pure N₂. In various embodiments as otherwise described herein, the purge gas comprises no more than 5 vol% H₂ (e.g., no more than 1 vol% H₂, or 0.1 vol% H₂, or 0.01 vol% H₂, or substantially no H₂). The purging is performed for an time sufficient to substantially remove any undesirable reactants. For example, in various embodiments as otherwise described herein the purging may be performed for a time in the range of 0.1 hr to 24 hr, for example, in the range of 0.5 hr to 24 hr, or 1 hr to 24 hr, or 1 hr to 12 hr, or 1 hr to 6 hr.

The purging step is operated above ambient pressure in order to efficiently drive out undesired chemical species. The purge step also serves as an initial pressurization step for the catalyst in preparation for contact with syngas. Accordingly, the purging step (ii) is operated at a pressure in the range of 2 barg to 15 barg (e.g., in the range of 2 barg to 12 barg, or 2 barg to 10 barg, or 3 barg to 12 barg, or 3 barg to 10 barg, or 4 barg to 12 barg, or 4 barg to 10 barg,).

After the purge step (ii), the catalyst is contacted in the reaction zone with a gaseous reaction mixture that includes both H₂ and CO in a H₂:CO volume ratio in the range of 1:1 to 3:1. The person of ordinary skill in the art will select a desired gaseous reaction mixture source, based especially on the H₂:CO volume ratio desired for use in the ultimate FT synthesis process in the steady state. For example, in various embodiments as otherwise described herein, the gaseous reaction mixture comprises H₂ and CO in a H₂:CO volume ratio in the range of 1:1 to 2.5:1, or 1:1 to 2:1, or 1.5:1 to 3:1, or 1.5:1 to 2.5:1, or 1.5:1 to 2:1, or, 2:1 to 3:1, or 2:1 to 3:1. In various embodiments as otherwise described herein, the gaseous reaction mixture comprises H₂ and CO in a H₂:CO volume ratio in the range of 1.5:1 to 2.5:1.

The gaseous reaction mixture may also include other gases. For example, the gaseous reaction mixture may include carbon dioxide in an amount of no more than 20%, e.g., no more than 10%, or no more than 5%.

Advantageously, the process as described herein allows the utilization of relatively high proportions of H₂ and CO in the gaseous reaction feed at startup. In various embodiments as otherwise described herein, the gaseous reaction mixture comprises at least 20 vol% H₂ and CO (i.e., the proportion of H₂ and CO together is at least 20 vol%). For example, in various embodiments as otherwise described herein, the gaseous reaction mixture comprises at least 30 vol% H₂ and CO (e.g., at least 35 vol% H₂ and CO). In various embodiments as otherwise described herein, the gaseous reaction mixture comprises at least 40% vol% H₂ and CO (e.g., at least 45% H₂ and CO).

Nitrogen gas can be used as an inert gas in the gaseous reaction mixture. In certain embodiments as otherwise described herein, the gaseous reaction mixture further comprises N₂ in an amount up to 80 vol%, e.g., up to 70 vol%, or up to 60 vol%, or up to 50 vol%. For example, in particular embodiments, the gaseous reaction mixture comprises N₂ in an amount in the range of 20 vol% to 80 vol%, e.g., 20 vol% to 70 vol%, or 20 vol% to 60 vol%, or 30 vol% to 80 vol%, or 30 vol% to 70 vol%, or 30% to 60%, or 40 vol% to 80 vol%, or 40 vol% to 70 vol%, or 40 vol% to 60 vol%.

The total amount of H₂, CO and N₂ in the gaseous reaction mixture of processes as otherwise described herein is desirably high, e.g., at least 80 vol%, or at least 90 vol%, or at least 95 vol%, or at least 99 vol%.

Advantageously, the processes as described herein can avoid the need to slowly ramp the concentration of CO and H₂ within the gaseous reaction mixture. Accordingly, in certain embodiments as otherwise described herein, the combined proportion of H₂ and CO in the gaseous reaction mixture is not varied much throughout the process. For example, in various embodiments as otherwise described herein, the combined proportion of H₂ and CO does not vary by more than 30% throughout steps (iii)-(v), e.g., by more than 20%, or by more than 10%.

The gaseous reaction mixture may also comprise other gaseous components, such water, methane and other saturated and/or unsaturated light hydrocarbons, preferably being present at a combined concentration of less than 30% by volume.

It is often useful to increase reactor efficiency through recycling unreacted syngas and/or incompletely reacted products, such as light hydrocarbons, to the reactor. This technique is especially useful for the processes as described herein, which, in certain embodiments, include contacting a catalyst with H₂ and CO at temperatures below that where significant Fischer-Tropsch reaction takes place. Accordingly, in various embodiments as otherwise described herein, step (iii) further includes withdrawing a recycle stream from the reaction zone, and recycling at least a portion of the recycle stream to the reaction zone.

The contacting of step (iii) as otherwise described herein is performed at relatively low pressure compared to steady-state Fischer-Tropsch operation. Accordingly, in various embodiments as otherwise described herein, the contacting of step (iii) is performed at a pressure in the range of 5 barg to 20 barg. For example, in various such embodiments, the contacting is performed at a pressure in the range of 5 barg to 15 barg, or 5 barg to 12 barg, or 5 barg to 10 barg, or 10 barg to 20 barg, or 10 barg to 15 barg, or 12 barg to 20 barg, or 15 barg to 20 barg.

The processes described herein can be performed with high throughputs. For example, in various embodiments as otherwise described herein, the contacting of step (iii) is conducted at a GHSV in the range of 2000 hr⁻¹ to 12,000 hr⁻¹. For example, in various such embodiments the contacting is conducted at a GHSV in the range of 4000 hr⁻¹ to 12,000 hr⁻¹, or 6000 hr⁻¹ to 12,000 hr⁻¹, or 8000 hr⁻¹ to 12,000 hr⁻¹, or 2000 hr⁻¹ to 10,000 hr⁻¹, or 4000 hr⁻¹ to 10,000 hr⁻¹, or 6000 hr⁻¹ to 10,000 hr⁻¹, or 2000 hr⁻¹ to 8,000 hr⁻¹, or 4000 hr⁻¹ to 8,000 hr⁻¹, or 2000 hr⁻¹ to 6000 hr⁻¹.

The contacting of step (iii) as otherwise described herein is conducted at a relatively low temperature, no more than 140 °C, e.g., no more than 130 C or no more than 120 °C. In various embodiments as otherwise described herein, the contacting of step (iii) is performed at a temperature in the range of 80 °C to 140 °C. For example, in various particular such embodiments, the contacting of step (iii) is performed at a temperature in the range of 80 °C to 130 °C, or 80 °C to 120 °C. In various particular such embodiments, the contacting of step (iii) is performed at a temperature in the range of 100 °C to 140 °C, for example 100 °C to 130 °C, or 100 °C to 120 °C. In various particular such embodiments, the contacting of step (iii) is performed at a temperature in the range of 80 °C to 160 °C, for example 110 °C to 130 °C. The contacting of step (iii) will typically be performed at a temperature that is near the temperature of step (i), e.g., with in 10 °C of such temperature.

While in contact with the gaseous reaction mixture, the reaction zone is then heated to a temperature of at least 200 °C, and pressurized to a pressure in the range of 25 barg to 45 barg. Under these conditions, steady state Fischer-Tropsch synthesis can be achieved. The heating of step (iv) and the pressurization of step (v) can be performed in any time sequence, including simultaneously. The temperature and pressure can be selected to be the temperature and pressure of the steady-state FT process to be performed.

Without wishing to be bound by theory, the present inventors have determined that heating the reaction zone in the presence of syngas comprising H₂ and CO and a freshly reduced catalyst can result in increasing Fischer-Tropsch synthesis activity as the temperature is raised. In turn, an increased synthesis rate will result in increased heat generated from the exothermic Fischer-Tropsch synthesis reaction, the temperature must be carefully increased to prevent a dangerous thermal runaway. Accordingly, in various embodiments as otherwise described herein, the heating step (iv) is conducted with a plurality of different heating rates, comprising:
(a) heating at a rate in the range of 10 °C/hr to 60 °C/hr for temperatures below 160 °C;
(b) heating at a rate in the range of 2 °C/hr to 30 °C/hr for temperatures in the range of 160 °C to 180 °C;
(c) heating at a rate in the range of 0.5 °C/hr to 7 °C/hr for temperatures in the range of 180 °C to 220 °C.

In various embodiments as otherwise described herein, heating rate condition (a) comprises heating at a rate in the range of 10 °C/hr to 45 °C/hr, or 10 °C/hr to 30 °C/hr, or 20 °C/hr to 60 °C/hr, or 20 °C/hr to 45 °C/hr. In various embodiments as otherwise described herein, heating rate condition (b) comprises heating at a rate in the range of 2 °C/hr to 20 °C/hr, or 2 °C/hr to 15 °C/hr, or 4 °C/hr to 30 °C/hr, or 4 °C/hr to 20 °C/hr, or 4 °C/hr to 15 °C/hr. In various embodiments as otherwise described herein, heating rate condition (c) comprises heating at a rate in the range of.0.5 °C/hr to 5 °C/hr, or 0.5 °C/hr to 4 °C/hr, or 1 °C/hr to 7 °C/hr, or 1 °C/hr to 5 °C/hr, or 1 °C/hr to 4 °C/hr, or 2 °C/hr to 7 °C/hr, or 2 °C/hr to 5 °C/hr, or 2 °C/hr to 4 °C/hr.

Upon reaching a temperature at which the Fischer-Tropsch reaction begins (typically around 150-180 °C), a hydrocarbon product composition will begin to form in the reaction zone. And as the Fischer-Tropsch reaches its steady-state operation (e.g., under the conditions of steps (iv) and (v)), hydrocarbon product composition will continue to form, beneficially providing desired products. Accordingly, in various embodiments as otherwise described herein, the process further comprises, after steps (iv) and (v):
(vi) performing a Fischer-Tropsch synthesis and withdrawing a hydrocarbon product composition from the reaction zone, wherein the hydrocarbon composition comprises one or more of methane, C₅₊ hydrocarbons, and oxygenates.

In various embodiments of the the temperature ramp in step (iii) or step (iv) may include dwells or holds in the ramping temperature to allow for catalyst stabilisation, catalyst conditioning or wax/hydrocarbon buildup in the catalyst pores.

Subject to the limitations described herein, the person of ordinary skill in the art can adapt conventional Fischer-Tropsch processes to arrive at the processes of the disclosure. For example, the temperature of the Fischer-Tropsch synthesis can suitably be in the range of 200-400 °C, such as 200-300 °C, or 210-400 °C, or 210-300 °C, or 220-400 °C, or 220-300 °C. In various embodiments as otherwise described herein, the temperature of the Fischer-Tropsch synthesis is in the range of 200-250 °C, e.g., or 200-240 °C, or 200-230 °C, or 200-220 °C, or 210-250 °C, or 210-240 °C, or 210-230 °C, or 220-250 °C, or 220-240 °C, or 230-250 °C. The pressure of the reaction may suitably be in the range from 25-45 barg, e.g., 30-40 barg, or 30-35 barg, or 35-45 barg, or 35-40 barg, or 40-45 barg.

The Fischer-Tropsch synthesis reaction may be performed in any suitable type of reactor, for example it may be performed in a fixed bed reactor, a slurry bed reactor, or a CANS^{™} reactor. Specifically with respect to **CANS** reactors, a passivated catalyst material as described herein can be packaged in a modular catalyst container suitable for use in a reactor tube of the **CANS** reactor (e.g., offsite then transported to the reactor site).

The hydrocarbon composition can vary based on changes in process conditions as known in the art. In certain embodiments, the hydrocarbon composition comprises hydrocarbons (e.g., linear hydrocarbons, branched hydrocarbons, saturated or unsaturated hydrocarbons) and oxygenated derivatives thereof. Examples of the oxygenated derivatives thereof include hydrocarbons with one or more functional group of alcohols, aldehydes, ketones, carboxylic acids, esters, and combinations thereof. In certain embodiments as otherwise described herein, the hydrocarbon composition comprises at least one of alkanes, alkenes, and alcohols.

The person of ordinary skill in the art will adapt suitable catalyst materials for use in the processes described herein. In various desirable embodiments of the processes described herein, the catalyst includes cobalt and optionally one or more other metals or reaction modifiers disposed on a support. Supported cobalt-based materials are well-known in the art, and can generally be adapted for use in the processes and materials described herein.

In various embodiments as otherwise described herein, the catalyst materials as described herein include cobalt in the range of 5 wt% to 35 wt%, calculated as cobalt(0). For example, in certain embodiments as otherwise described herein, cobalt may be present in the range of 7-35 wt%, or 10-35 wt%, or 5-25 wt%, or 7-25 wt%, or 10-25 wt%, or 5-20 wt%, or 7-20 wt%, or 10-20 wt%.

The catalyst materials as described herein can include other metal species, e.g., as promoters. For example, in certain embodiments as otherwise described herein, a catalyst material includes manganese, for example, in an amount in a range of no more than 15 wt%, e.g., no more than 12 wt%, or no more than 10 wt%, or no more than 7 wt%, calculated as manganese(0). In certain such embodiments, a catalyst material includes manganese in an amount in the range of 0.1-15 wt%, e.g., 0.5-12 wt%, or 0.5-10 wt%. Of course, in other embodiments substantially no manganese is present (e.g., less than 0.1 wt% or less than 0.5 wt%) manganese is present.

Various support materials are known in the art and may be selected based on the precise requirements of the FT reactor or other chemical, mechanical or economic requirements. In certain embodiments as otherwise described herein, the support comprises at least one of titanium oxide, zirconium oxide, cerium oxide, aluminum oxide, silicon oxide and zinc oxide. In particular embodiments, the support comprises exactly one of titanium oxide, zirconium oxide, cerium oxide, aluminum oxide, silicon oxide and zinc oxide.

The catalyst can be prepared using methods conventional in the art. In certain embodiments, the cobalt is introduced onto the support through the introduction of a solution containing a soluble cobalt salt (e.g., cobalt nitrate) to the support and the combination calcined and/or oxidized, rendering insoluble cobalt particles (e.g., as cobalt oxide) on the support. In certain embodiments, the first catalyst material comprises the combination of the calcined metal (e.g., including calcined cobalt) adhered to the support. In particular embodiments, cobalt of the first catalyst material (i.e., as least a portion of the cobalt, up to the entirety of the cobalt, for example, at least 50%, at least 75%, or at least 90%) is in the form of at least one of cobalt oxide and cobalt hydroxide. For example, the cobalt may be cobalt oxide (e.g., CoO, Co₃O₄, or Co₂O₃, or a combination thereof) or cobalt hydroxide (e.g., Co(OH)₂ or Co(OH)₃ or a combination thereof), or a combination of cobalt oxide and cobalt hydroxide.

In certain embodiments, the catalyst must be substantially reduced to generate the reduced catalyst material. This process results in at least portion of the cobalt being transformed into cobalt metal. Desirably, the reduction results in at least 50% of the cobalt being provided as cobalt(0), e.g., at least 75%, or at least 90% of the cobalt being provided as cobalt(0). The person of ordinary skill in the art can use conventional methods to reduce cobalt catalyst materials (e.g., cobalt oxide- or cobalt hydroxide-based materials) to metallic form. In certain embodiments as otherwise described herein, the first reducing agent is hydrogen gas, H₂. The hydrogen gas may be mixed with other gases, such as an inert carrier gas. Examples of such inert carrier gasses include nitrogen, carbon dioxide, argon, or helium. The hydrogen gas may also be mixed with carbon monoxide, with or without one or more additional carrier gasses. In certain embodiments, the reduction is effected by contacting the first catalyst material with a first reducing gas, wherein the first reducing gas comprises the first reducing agent, wherein the first reducing gas comprises at least 50 vol% H₂ (e.g., at least 60 vol%, or at least 70 vol%, or at least 80 vol%, or at least 90 vol%, or at least 95 vol%, or essentially 100 vol% H₂).

The reduction of the catalyst material to provide the reduced catalyst material is performed at a first temperature. In certain embodiments as otherwise described herein, the first temperature is in the range of 200 °C to 400 °C. For example, in certain embodiments, the first temperature is in the range of 250 °C to 350 °C, or in the range of 260 °C to 340 °C, or in the range of 270 °C to 330 °C, or in the range of 280 °C to 320 °C, or in the range of 290 °C to 310 °C. In certain embodiments, the first temperature is approximately 300 °C. The reduction of the first catalyst material to the reduced catalyst material occurs at a first pressure. In certain embodiments as otherwise described herein, the first pressure is in the range of 0.5 bara to 5 bara, e.g., 0.7 bara to 3 bara. The reduction can be performed for a time (e.g., up to 48 hours, e.g., 2-48 hours or 8-30 hours) and under conditions sufficient to provide the desired degree of reduction as described above.

As described herein, the treatment of the first catalyst material with the first reducing agent produces a reduced catalyst material that includes cobalt as cobalt metal (e.g., in an amount of at least 50%, e.g., at least 75%, or at least 90% of the cobalt as described above).

As described herein, the catalyst performance is monitored through performance parameters such as COₓ conversion, C5+ Selectivity, CH4 Selectivity, and C5+ Productivity. These are calculated based on per pass conversion as well as total carbon conversion. Conversion is maintained by adjusting applied temperature (from pressure level of steam drum) and increased gradually to maintain conversion over the life of the catalyst.

### EXAMPLES

The Examples that follow are illustrative of specific embodiments of the methods of the disclosure, and various uses thereof. They are set forth for explanatory purposes only, and are not to be taken as limiting the scope of the disclosure.

### Example 1

A Fischer-Tropsch reactor was charged with a catalyst comprising 10% Co and 1%Mn on TiO₂. The catalyst was dried at 7 barg and 165 °C under N₂ with a GHSV of 2000 hr⁻¹. Next, the catalyst was heated to 250 °C at 10 °C/hr and activated at 26 barg with 50% H₂ in N₂ at a GHSV of 6700 hr⁻¹ for 24 hours to produce a reduced catalyst.

The reduced catalyst was then cooled under a mixture of H₂ and N₂ to a temperature of 130 °C in preparation for the startup procedure. Subsequently, a gaseous reaction mixture comprising H₂ and CO in a ratio of 1.8:1 admixed with 51% N₂ was introduced at a GHSV of 8795 hr⁻¹. The reactor was heated from to 150 °C at 50 °C/hr, then from 150 °C to 160 °C at 25 °C/hr, then from 160 °C to 180 °C at 5 °C/hr, and finally from 180 °C to 205 °C at 2 °C/hr. The pressure was then increased to 30 barg.

The temperature was then maintained at 205 °C for 200 hr:

| Temperature | 205 °C |
|---|---|
| CO Conversion | 22.2% |
| CH₄ selectivity | 4.4% |
| C₅₊ selectivity | 89.7% |

Subsequently, the temperature was raised to 212 °C and Fischer-Tropsch synthesis continued for 280 hr to increase conversion to the experimental target:

| Temperature | 212 °C |
|---|---|
| CO Conversion | 35.0% |
| CH₄ selectivity | 5.7% |
| C₅₊ selectivity | 88.2% |

### Comparative Example 1

In this Comparative Example, the catalyst was reduced as in Example 1 but subsequently cooled to only 180 °C. A gaseous reaction mixture comprising H₂ and CO in a ratio of 1.8:1 admixed with 95 % N₂ was then introduced, as inclusion of less N₂ resulting in a dangerously exothermic reaction at this temperature.

Various exemplary embodiments of the disclosure include, but are not limited to the enumerated embodiments of the claims as listed below, which can be combined in any number and in any combination that is not technically or logically inconsistent.

Embodiment 1. A process for the initiation of Fischer-Tropsch synthesis in a reaction zone having a catalyst disposed therein, the catalyst being in a reduced form, the process comprising:
(i) providing the reaction zone with a temperature of no more than 160 °C; then
(ii) purging the reaction zone at a temperature of no more than 160 °C with a purge gas comprising N₂, at a pressure in the range of 2 barg to 15 barg; then
(iii) contacting the catalyst in the reaction zone with a gaseous reaction mixture comprising H₂ and CO in a ratio of in the range of 1:1 to 3:1 at a pressure of no more than 45 barg and at a temperature of no more than 160 °C; then, while in contact with the gaseous reaction mixture,
(iv) heating the reaction zone to a temperature of at least 200 °C; and
(v) pressurizing the reaction zone to a pressure in the range of 25 barg to 45 barg.

Embodiment 2. The process of embodiment 1 wherein the reaction zone in step (i) is at a temperature in the range of 100 °C to 140 °C.

Embodiment 3. The process of any of embodiments 1-2, wherein the purge gas includes at least 80 vol% N₂, e.g., at least 90 vol% N₂, or at least 95 vol% N₂.

Embodiment 4. The process of any of embodiments 1-3, wherein the purge gas comprises no more than 5% H₂.

Embodiment 5. The process of any of embodiments 1-4, wherein the purge gas includes no more than 1% H₂, e.g., no more than 0.1% H₂, or no more than 0.01% H₂, or substantially no H₂.

Embodiment 6. The process of any of embodiments 1-5, wherein the purging step (ii) is conducted for a time in the range of 0.1 hr to 24 hr.

Embodiment 7. The process of any of embodiments 1-6, wherein H₂ and CO together are present in the gaseous reaction mixture in an amount in the range of 10% to 80 vol% (e.g., in the range of 20 vol% to 70 vol%, or in the range of 30 vol% to 70 vol%).

Embodiment 8. The process of any of embodiments 1-7, wherein H₂ and CO are present in the gaseous reaction mixture in a H₂:CO ratio in the range of 1:1 to 2.5:1, or 1:1 to 2:1, or 1.5:1 to 3:1, or 1.5:1 to 2.5:1, or 1.5:1 to 2:1, or, 2:1 to 3:1, or 2:1 to 3:1.

Embodiment 9. The process of any of embodiments 1-8, wherein H₂ and CO are present in the gaseous reaction mixture in a H₂:CO ratio in the range of 1.5:1 to 2.5:1.

Embodiment 10. The process of any of embodiments 1-9, wherein the gaseous reaction mixture comprises at least 20 vol% H₂ and CO.

Embodiment 11. The process of any of embodiments 1-10, wherein the gaseous reaction mixture comprises at least 30 vol% H₂ and CO (e.g., at least 35 vol%, or at least 40 vol%, or at least 45 vol%).

Embodiment 12. The process of any of embodiments 1-11, wherein the gaseous reaction mixture further comprises inert species in an amount up to 80 vol%, e.g., up to 70 vol%, or up to 60 vol%, or up to 50 vol%.

Embodiment 13. The process of any of embodiments 1-12, wherein the gaseous reaction mixture comprises N₂ in an amount in the range of 20 vol% to 80 vol%, e.g., 20 vol% to 70 vol%, or 20 vol% to 60 vol%, or 30 vol% to 80 vol%, or 30 vol% to 70 vol%, or 30% to 60%, or 40 vol% to 80 vol%, or 40 vol% to 75 vol%, or 40 vol% to 70 vol%.

Embodiment 14. The process of any of embodiments 1-13, wherein the total amount of reactive species (H₂, CO, olefins)in the gaseous reaction mixture is at least 20 vol%, e.g., at least 30 vol%, or at least 35 vol%, or at least 40 vol%.

Embodiment 15. The process of any of embodiments 1-14, wherein the combined proportion of H₂ and CO does not vary by more than 30% throughout steps (iii)-(v), e.g., by more than 20%, or by more than 10%.

Embodiment 16. The process of any of embodiments 1-15, wherein step (iii) further comprises withdrawing a recycle stream comprising syngas from the reaction zone, and recycling at least a portion of the recycle stream to the reaction zone.

Embodiment 17. The process of any of embodiments 1-16, wherein step (iii) is conducted at a pressure in the range of 5 barg to 20 barg, e.g., 5 barg to 15 barg, or 5 barg to 12 barg, or 5 barg to 10 barg, or 10 barg to 20 barg, or 10 barg to 15 barg, or 12 barg to 20 barg, or 15 barg to 20 barg.

Embodiment 18. The process of any of embodiments 1-17, wherein the gaseous reaction mixture is contacted at a GHSV in the range of 2000 hr⁻¹ to 12,000 hr⁻¹, e.g., in the range of 4000 hr⁻¹ to 12,000 hr⁻¹, or 6000 hr⁻¹ to 12,000 hr⁻¹, or 8000 hr⁻¹ to 12,000 hr⁻¹, or 2000 hr⁻¹ to 10,000 hr⁻¹, or 4000 hr⁻¹ to 10,000 hr⁻¹, or 6000 hr⁻¹ to 10,000 hr⁻¹, or 2000 hr⁻¹ to 8,000 hr⁻¹, or 4000 hr⁻¹ to 8,000 hr⁻¹, or 2000 hr⁻¹ to 6000 hr⁻¹.

Embodiment 19. The process of any of embodiments 1-18, wherein the heating of step (iv) is conducted at a plurality of heating rate conditions, comprising:
(a) heating at a rate in the range of 10 °C/hr to 60 °C/hr for temperatures below 160 °C;
(b) heating at a rate in the range of 2 °C/hr to 30 °C/hr for temperatures in the range of 160 °C to 180 °C;
(c) heating at a rate in the range of 0.5 °C/hr to 7 °C/hr for temperatures in the range of 180 °C to 220 °C.

Embodiment 20. The process of any of embodiments 1-19, wherein heating rate condition (a) comprises heating at a rate in the range of 10 °C/hr to 45 °C/hr, or 10 °C/hr to 30 °C/hr, or 20 °C/hr to 60 °C/hr, or 20 °C/hr to 45 °C/hr.

Embodiment 21. The process of any of embodiments 1-20, wherein heating rate condition (a) comprises heating at a rate in the range of 2 °C/hr to 20 °C/hr, or 2 °C/hr to 15 °C/hr, or 4 °C/hr to 30 °C/hr, or 4 °C/hr to 20 °C/hr, or 4 °C/hr to 15 °C/hr.

Embodiment 22. The process of any of embodiments 1-21, wherein heating rate condition (a) comprises heating at a rate in the range of.0.5 °C/hr to 5 °C/hr, or 0.5 °C/hr to 4 °C/hr, or 1 °C/hr to 7 °C/hr, or 1 °C/hr to 5 °C/hr, or 1 °C/hr to 4 °C/hr, or 2 °C/hr to 7 °C/hr, or 2 °C/hr to 5 °C/hr, or 2 °C/hr to 4 °C/hr.

Embodiment 23. The process of any of embodiments 1-22, further comprising, after steps (iv) and (v):
(vi) performing a Fischer-Tropsch synthesis at a temperature of at least 200 C and a pressure in the range of 30-45 barg, and withdrawing a hydrocarbon product composition from the reaction zone, wherein the hydrocarbon composition comprises one or more of methane, C₅₊ hydrocarbons, and oxygenates.

Embodiment 24. The process of embodiment 23, wherein the Fischer-Tropsch synthesis is performed at a temperature in the range of 200-250 °C, e.g., or 200-240 °C, or 200-230 °C, or 200-220 °C, or 210-250 °C, or 210-240 °C, or 210-230 °C, or 220-250 °C, or 220-240 °C, or 230-250 °C.

Embodiment 25. The process of embodiment 23 or embodiment 24, wherein the Fischer-Tropsch synthesis is performed at a pressure in the range of 30-45 barg, e.g., 30-40 barg, or 30-35 barg, or 35-45 barg, or 35-40 barg, or 40-45 barg.

Embodiment 26. The process of any of embodiments 1-25, wherein the catalyst comprises cobalt disposed on a support.

Embodiment 27. The process of embodiment 26, wherein the support comprises at least one of titanium oxide, zirconium oxide, cerium oxide, aluminum oxide, silicon oxide and zinc oxide.

Embodiment 28. The process of embodiment 27 wherein the support is a titanium oxide support.

Embodiment 29. The process of any of embodiments 26-28, wherein the catalyst comprises 5-35 wt% cobalt, calculated as cobalt(0).

Embodiment 30. The process of any of embodiments 26-29, wherein the catalyst comprises cobalt in an amount in the range of 7-35 wt%, or 10-35 wt%, or 5-25 wt%, or 7-25 wt%, or 10-25 wt%, or 5-20 wt%, or 7-20 wt%, or 10-20 wt%, calculated as cobalt(0).

Embodiment 31. The process of any of embodiments 26-30, wherein the catalyst comprises 0.1-15 wt% manganese, e.g., 0.1-15 wt%, e.g., 0.5-12 wt%, or 0.5-10 wt%, calculated as manganese(0).

Embodiment 32. The process of any preceding embodiments wherein at least some hydrocarbon product is removed from the exit stream of the reactor and a portion of the gas phase product and unreacted syngas is recycled back to the inlet of the reactor.

The particulars shown herein are by way of example and for purposes of illustrative discussion of certain embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for the initiation of Fischer-Tropsch synthesis in a reaction zone having a catalyst disposed therein, the catalyst being in a reduced form, the process comprising:
(i) providing the reaction zone with a temperature of no more than 160 °C; then
(ii) purging the reaction zone at a temperature of no more than 160 °C with a purge gas comprising N₂, at a pressure in the range of 2 barg to 15 barg; then
(iii) contacting the catalyst in the reaction zone with a gaseous reaction mixture comprising H₂ and CO in a ratio of in the range of 1:1 to 3:1 at a pressure of no more than 45 barg and at a temperature of no more than 160 °C; then, while in contact with the gaseous reaction mixture,
(iv) heating the reaction zone to a temperature of at least 200 °C; and
(v) pressurizing the reaction zone to a pressure in the range of 25 barg to 45 barg.

2. The process of claim 1 wherein the reaction zone in step (i) is at a temperature in the range of 100 °C to 140 °C.

3. The process of any of claim 1 or claim 2, wherein the purge gas comprises no more than 5% H₂.

4. The process of any of claims 1-3, wherein H₂ and CO together are present in the gaseous reaction mixture in an amount in the range of 10% to 80 vol% (e.g., in the range of 20 vol% to 70 vol%, or in the range of 30 vol% to 70 vol%).

5. The process of any of claims 1-4, wherein H₂ and CO are present in the gaseous reaction mixture in a H₂:CO ratio in the range of 1:1 to 2.5:1, or 1:1 to 2:1, or 1.5:1 to 3:1, or 1.5:1 to 2.5:1, or 1.5:1 to 2:1, or, 2:1 to 3:1, or 2:1 to 3:1.

6. The process of any of claims 1-5, wherein the total amount of reactive species (H₂, CO, olefins)in the gaseous reaction mixture is at least 20 vol%, e.g., at least 30 vol%, or at least 35 vol%, or at least 40 vol%.

7. The process of any of claims 1-6, wherein step (iii) further comprises withdrawing a recycle stream comprising syngas from the reaction zone, and recycling at least a portion of the recycle stream to the reaction zone.

8. The process of any of claims 1-7, wherein the heating of step (iv) is conducted at a plurality of heating rate conditions, comprising:
(a) heating at a rate in the range of 10 °C/hr to 60 °C/hr for temperatures below 160 °C;
(b) heating at a rate in the range of 2 °C/hr to 30 °C/hr for temperatures in the range of 160 °C to 180 °C;
(c) heating at a rate in the range of 0.5 °C/hr to 7 °C/hr for temperatures in the range of 180 °C to 220 °C.

9. The process of any of claims 1-8, further comprising, after steps (iv) and (v):
(vi) performing a Fischer-Tropsch synthesis at a temperature of at least 200 °C and a pressure in the range of 30-45 barg, and withdrawing a hydrocarbon product composition from the reaction zone, wherein the hydrocarbon composition comprises one or more of methane, C₅₊ hydrocarbons, and oxygenates.

10. The process of any of claims 1-9, wherein the catalyst comprises cobalt disposed on a support.

11. The process of claim 10, wherein the support comprises at least one of titanium oxide, zirconium oxide, cerium oxide, aluminum oxide, silicon oxide and zinc oxide.

12. The process of claim 11 wherein the support is a titanium oxide support.

13. The process of any of claims 10-12, wherein the catalyst comprises 5-35 wt% cobalt, calculated as cobalt(0).

14. The process of any of claims 10-13, wherein the catalyst comprises 0.1-15 wt% manganese, e.g., 0.1-15 wt%, e.g., 0.5-12 wt%, or 0.5-10 wt%, calculated as manganese(0).

15. The process of any preceding claims wherein at least some hydrocarbon product is removed from the exit stream of the reactor and a portion of the gas phase product and unreacted syngas is recycled back to the inlet of the reactor.
